# EUROPEAN PATENT SPECIFICATION

(11) **EP 4 018 987 B1**
(45) Date of publication and mention of the grant of the patent: **23.04.2025**
(21) Application number: 21215698.8
(22) Date of filing: 17.12.2021
(51) Int. Cl.: A61F 13/15, A61F 13/49, B32B 5/02, B32B 27/12

(54) **STRETCH-BONDED LAMINATE AND METHOD TO PRODUCE A MACHINE DIRECTION STRETCH-BONDED LAMINATE**
STRECKGEBUNDENES LAMINAT UND VERFAHREN ZUR HERSTELLUNG EINES LAMINATS DURCH MASCHINENDIREKTIONALES DEHNUNGSVERBINDEN
STRATIFIÉ LIÉ PAR EXTENSION ET PROCÉDÉ DE PRODUCTION D'UN STRATIFIÉ À ÉTIRAGE DIRECTIONNEL DE MACHINE

(30) Priority: 23.12.2020 CN 202011536603
(43) Date of publication of application: 29.06.2022
(73) Proprietor: Nitto Advanced Film Gronau GmbH, 48599 Gronau (DE)
(72) Inventor: Trinkaus, Jan Michael, 53881 Euskirchen (DE); Gengyi, Lin, Taicang, 215400 (CN)
(74) Representative: Andrejewski - Honke Patent- und Rechtsanwälte Partnerschaft mbB

(56) References cited:
- EP-A1- 3 689 307
- EP-B1- 3 082 702
- EP-B1- 3 083 247
- WO-A1-2012/036599
- US-A1- 2020 383 841
- US-B2- 6 843 872

## Description

The present invention relates to a stretch-bonded laminate with an elastic film and at least one first nonwoven web comprising a stretch direction along which the first nonwoven web is gathered due to the elastic relaxation of the elastic film. The stretch-bonded laminate is preferably obtained by stretching along a machine direction. Thus the present invention is also directed to a method to produce a machine direction stretch-bonded laminate.

The stretch-bonded laminate is especially provided for personal care products like baby diapers and training pants. Such personal care products are provided for a single use. Thus, low costs for the material and the manufacturing as well as a low consumption of resources are aspired. The elastic stretch-bonded laminate can be incorporated in the personal care products as an elastic member in the hip region to keep the personal care product in a close and preferably fluid tight contact to the user. In addition the wearing comfort is essential, where any irritation or impression of the skin must be avoided. Accordingly it is necessary to adjust the elastic properties of the laminate carefully.

In case of a direct contact to a wearer's skin at least one first nonwoven web can provide a soft surface of the stretch-bonded laminate in case that it is in direct skin contact. To achieve good mechanical properties as well as a pleasant appearance, the elastic film can be provided between said first nonwoven web and a second nonwoven web. With respect to stretch-bonded laminates it is known that the first nonwoven web and the optionally provided second nonwoven web is/are gathered due to the elastic relaxation of the elastic film. Due to the elastic relaxation and the gathering of the nonwoven, a very soft and bulky structure with a textile appearance is achieved.

Due to the stretch-bonding, the laminate can be stretched against the elastic forces of the elastic film until a predetermined threshold stretching rate is reached which is corresponding to the stretching of the elastic film during lamination. When reaching this predetermined threshold stretching rate the first nonwoven web lies flat on the elastic film without or only little deformation of its initial structure. Usually the threshold stretching rate is noticed by a user as a steep increase of the force necessary for further stretching due to the plastic deformation of the nonwoven, wherein the arrangement of the fibres in the nonwoven is changed and/or the single fibres are plastically stretched.

As earlier mentioned, elastic laminates for use in personal care products require a delicate balance of elastic force required to keep the product in position and the pressure exerted on the body. Too high elastic forces and resulting high pressure lead to discomfort and pressure marks. Furthermore, a certain level of breathability is required to avoid hydratisation of the skin underneath.

A cheap way to achieve breathability is via elastic strands between two layers of nonwoven. This approach is especially used for pant-type baby diapers like training pants. However, thin elastic strands often leave pressure markings because the elastic force is concentrated on the narrow contact area of the single elastic strands with the skin. In addition, strand laminates often cause large corrugations that add visible volume, which is a disadvantage when designing for discreetness in a product for grown-ups.

A better way to achieve breathability, discreetness, and even force distribution is through elastic film laminates that are perforated before and/or during the lamination process. This can especially be achieved through ultrasonic bonding.

Corresponding laminates are for example disclosed in US 4,842,596, EP2064052B1 and EP3251642A1.

As disclosed in WO 2012/036599 A1, EP 2 726 037 B1 and the above mentioned EP 3 251 642 A1, perforations can be provided at least on one side of ultrasonic-bonding points. Due to the tension of the elastic film during the stretch-bonding process and the local melting of the film material at the ultrasonic bonds the film locally tears and retracts. Like disclosed in the mentioned prior art, a perforation may occur only on one side or on both sides of each ultrasonic bond.

According to EP 3 689 310 A1 and WO 2019/065024 A1 it is also known to modulate the elastic properties of the laminate by a variation of the bonding pattern.

It is an object of the present invention to create a laminate with superior stretch properties that displays fabric-like appearance, while being breathable.

To achieve this object the present invention provides a stretch-bonded laminate according to claim 1.

According to the invention the first and second rows and the lines extending in the stretch direction are provided for different purposes.

The elongated structures are each attributed to one adjacent perforation. The perforations form weak spots so that the first rows do not significantly contribute to the elastic properties and the elastic recovery of the laminate. Accordingly, the elastic properties of the stretch-bonded laminate can be easily manipulated by the width of the first rows, i.e. the lengths of the elongated structure in relation to the width of the lines and the second rows respectively. In difference to a laminate with elastic strands the elastic film is also present in the first rows between the perforations so that the overall stability of the laminate is enhanced.

When the elongated structures are created by ultrasonic bonding according to a preferred embodiment of the invention the first nonwoven web is bonded to the elastic film at the elongated structures so that further strengthening of the material is achieved, wherein the bulkiness of the nonwoven web due to the elastic relaxation is restricted to the areas between the ultrasonic bonds as elongated structures.

The second rows are provided for the connection between the first nonwoven web and the elastic film, wherein the respective bonding areas are preferably generated by ultrasonic bonding. Especially the elongated structures and the bonding areas can be provided as an ultrasonic bonding pattern so that the bonding areas are in a certain extent similar to the elongated structures. As mentioned with respect to the elongated structures the ultrasonic bonding process can generate additional perforations joining the bonding areas, wherein such additional perforations can occur only on one side of the bonding areas or on both sides of the bonding areas with respect to the stretching direction.

Nevertheless the bonding areas of the second rows on the one hand and the elongated structures of the first rows on the other hand differ at least in their extension along the transverse direction. According to the invention the width of the first row determined by the lengths of the elongated structures is more than 1.5, preferably more than 2 of the widths of the second rows determined by the size of the bonding elements measured in transverse direction. The ratio may be between 1.5 and 4, especially between 2 and 3.

The first and second rows are separated by the lines extending in the stretch direction with the first nonwoven lying unconnected on the uninterrupted elastic film. At these lines the elastic properties of the film are not directly affected. Accordingly these lines are essential for the elastic recovery of the stretch-bonded laminate. It is clear that only by changing the width of the lines and the first and second rows the elastic properties can be adjusted in very broad range.

In principle a continuous pattern of elongated structures forming the first rows, bonding areas forming the second rows and lines in between can be provided.

As an alternative the width of the rows and the lines may vary in the stretch direction and/or the transverse direction to provide a further modification of the stretching properties. In case of such a further modification the alignment of the stretch-bonded laminate in a final product like a diaper or training pant must be considered.

The stretch-bonded laminate may have a first segment extending in the stretch direction with the first rows having the first width and a second segment extending in the stretch direction with first rows having a different second width.

As an alternative or in addition along the stretch direction sections with a repeating bonding pattern can be provided. For example the lengths of each section may correspond to the lengths of laminate at a diaper or a training pant. As an example an intermediate part of each section can be provided with the above-mentioned first and second rows and the lines in between, wherein edges with a different pattern can be provided. As an example connection regions with lower stretch ability might be provided at the edges by omitting the elongated structures or by providing the elongated structures with a reduced length.

As explained above especially the width of the first and second rows, the widths of the lines and the ratio of these parameters are essential for the stretch properties and the mechanical properties in general. According to the invention the distance between successive first rows corresponding to the widths of one second row and two lines is between 1 mm and 5 mm.

The widths of the second rows is preferably between 0.4 mm and 1.6 mm, especially between 0.6 mm and 1.2 mm.

With respect to the dimensions of the laminate along the stretch direction the elastic relaxation of the elastic film and the gathering of at least first nonwoven web must be considered.

Especially at the bonding areas and the elongated structures the elastic recovery is incomplete and less than the average recovery of the complete stretch-bonded laminate in a microscopic scale. Against this background the size of the bonding areas and the elongated structures is discussed below in reference to a predetermined threshold stretching rate at which the at least first nonwoven web lies flat on the elastic film without deformation of its initial structure, at least outside of the bonding areas and the elongated structures. The stretch-bonded laminates at the predetermined threshold stretching rate corresponds essentially to the stretching of the elastic film during lamination. The predetermined threshold stretching rate and thus the stretching of the elastic film during lamination is typically between 100% to 500% and especially between 200% and 400%.

Considering ultrasonic bonding the dimensions of the bonding areas and the elongated structures essentially correspond to the bonding pattern of the ultrasonic bonding process.

With respect to the stretched state of the laminate at the predetermined threshold stretching rate or the elastic film during lamination the bonding areas preferably comprise a regular shape with essentially the same dimensions along the stretch direction and the transverse direction. This criterion is for example met by cycles and squares with or without rounded corners.

The width of the first rows can be in between 1 mm and 5 mm, preferably between 1.5 mm and 3.5 mm. Referring to the bonding pattern and the stretched laminate the extension of the elongated structures along the stretch direction is less than the widths of the first rows. The extension of the bonding areas and the elongated structures can be for example in the range of 0.2 mm to 1 mm, preferably in the range between 0.4 mm and 0.8 mm.

The width of the lines and can be between 0.4 mm and 1.6 mm, preferably between 0.6 mm and 1 mm.

According to the invention the elongated structures and the bonding areas are together aligned along columns extending in the transverse direction. Accordingly a rectangular or essentially rectangular lattice structure is formed. The lattice structure is characterised by good mechanical properties and a pleasant appearance.

With respect to the predetermined stretching rates and thus the bonding pattern the area ratio of the elongated structures in comparison to the full laminate can be between 4% and 12%, preferably between 5.5% and 9%.

According to the invention the first rows and the second rows extend along the stretching direction. According to this feature both rows may also comprise partially or in general a direction component extending in cross direction but the stretching direction is in any case the predominant direction.

Referring to a general direction component extending in cross direction the rows may be slanted against the stretching direction especially with an angle of less than 25°.

According to an embodiment of the invention the first rows, the second rows and the lines extend straight in the stretch direction. The first and second rows may be exactly parallel to the stretching direction or slanted like mentioned above, epically slightly slanted.

As an alternative first rows, the second rows and the lines extend wavelike in the stretch direction with preferably a maximum deviation angle below 45°. The wavelike extension can be chosen especially under consideration of esthetic aspects without changing the fundamental features of the present invention. A straight centre axis of each wavelike row may be exactly parallel to the stretching direction or slanted like mentioned above, epically slightly slanted.

Especially when the stretch-bonded laminate is produced as an endless web in a continuous process the first and second rows or at least a straight centre axis of the rows are preferably parallel to the stretching direction. In a machine direction stretch bonding process using a regular repeating pattern on a bonding roll such an alignment may occur inevitable.

In addition the alignment of the first and second rows or at least a straight centre axis of the rows parallel to the stretching direction is believed to be advantageous with respect to the mechanical properties of the laminate.

To provide a soft surface on both sides of the stretch-bonded laminate the elastic film can be provided between the first nonwoven web and a second nonwoven web.

The present invention is also directed to a method to produce a machine direction stretch-bonded laminate as explained above, wherein a continuous web of an elastic film is transported and stretched along a machine direction with a stretching ratio between 150% and 500%, at least a first nonwoven web is transported along the machine direction, the elastic film and the first nonwoven web are laminated by ultrasonic bonding in a bonding pattern with the elastic film held in the stretched state, the bonding pattern comprises in an alternating sequence perpendicular to the machine direction along a cross direction first rows and second rows extending in the machine direction, said first rows comprise structures elongated along an angle of less than 5°, preferably less than 3° to the cross direction, due to the stretching of the elastic film and the ultrasonic bonding at least one perforation of the elastic film forms adjacent and to each elongated structure, wherein said second rows comprise bonding areas, wherein the first and second rows are separated by lines extending in the machine direction with the first nonwoven lying unconnected on the uninterrupted elastic film, the width of the first rows is more than 1.5 of the widths of the second rows measured in the cross direction and the distance between successive first rows is between 1 mm and 5 mm.

With respect to further embodiments of the method it is referred to the explanation of the stretch-bonded laminate with the machine direction corresponding to the stretch direction and the cross direction corresponding to the transverse direction.

The elastic film is usually extruded or coextruded and comprises a thermoplastic elastomer. As thermoplastic polymers styrolblockcopolymers (SBC) like SIS, SBS, SEBS, SEPS or preferably olefin-based elastic polymers (TPE-O) are provided, wherein the thermoplastic polymers might optionally blended with non-elastic polymers. For example olefin-based based elastic polymers might be blended with PP or PE. In addition blends of SBC and TPE-O with or without an admixture of a non-elastic polymer are also suitable. Furthermore processing aids, chemical stabilizers or other additives may be provided.

According to a preferred embodiment, the elastic film is provided as coextruded film with an elastic core and non-tacky surface layers. Preferred non-tacky surface layers can be based on polypropylene with anti-block additives or mineral fillers like talc or CaCo₃ Polypropylene can be provided as homopolymer or copolymer. In addition mixtures of PE and PP are well suitable for the non-tacky surface layers.

Referring to the relaxed state the thickness of the elastic film can be for example between 10 µm and 70 µm, especially between 15 µm and 50 µm.

The first nonwoven web and the optionally provided second nonwoven web can be embodied as single layer structure or a multilayer structure. Especially spunbond nonwoven, carded nonwoven and spunlace nonwoven are suitable for the first and second nonwoven web or at least a layer of the first and second nonwoven web. The nonwoven is preferably based on polypropylene.

To achieve a very peasant soft touch high softness spunbond nonwoven with a low internal bonding are known. In practice nonwoven webs with different layers like for example combined spunbond and meltblown layers or combined spunbond and carded layers are frequently used.

The nonwoven can as an option also comprise bi-component fibres with a symmetric or asymmetric arrangement of two polymer components. Referring to fibres of the nonwoven with a sheet-core-structure in combination with a coextruded elastic film comprising non-tacky surface layers, the sheet material of the fibres and the material of the non-tacky surface layers can matched to allow a reliable and solid bonding. Thus the same polymer or polymer group might be provided for the sheet material of the fibres and the material of the non-tacky surface layers respectively. As regards to a sheet-core-structure of the fibres it is preferred that the sheet comprises a lower melting temperature than the core.

In principle other polymeric or natural fibres may also be used for the nonwoven at least as an admixture. An admixture of fibres may be provided to amend the properties of the nonwoven like visual appetence, the surface feel or the affinity to water.

With respect to the overall costs of the stretch-bonded laminate a comparable low mass per unit area is preferred. With respect to the nonwoven material provided for lamination (i.e. before the gathering due to the elastic relaxation) the nonwoven may comprise a surface weight between 7 g/m² and 30 g/m², preferably between 10g/m² and 22 g/m².

The present invention is also directed to an absorbent article including a chassis comprising a topsheet, a backsheet, and an absorbent core disposed between the topsheet and the backsheet, and futher including as an elastic belt the stretch-bonded laminate referred to above.

Below the invention is described in reference to exemplary figures:
- Fig. 1: An arrangement to produce a machine direction stretch-bonded laminate;
- Fig. 2: An ultrasonic bonding pattern for the stretch-bonded laminate;
- Fig. 3: A schematic representation of the stretch-bonded laminate in the stretched state;
- Fig. 4: A cross section through the stretch-bonded laminate in the relaxed state;
- Fig. 5: An alternative embodiment of the bonding pattern shown in fig. 2;
- Fig. 6: A training pant for babies with a stretch-bonded laminate in a hip region;
- Fig. 7: Another alternative embodiment of the bonding pattern shown in fig. 1.

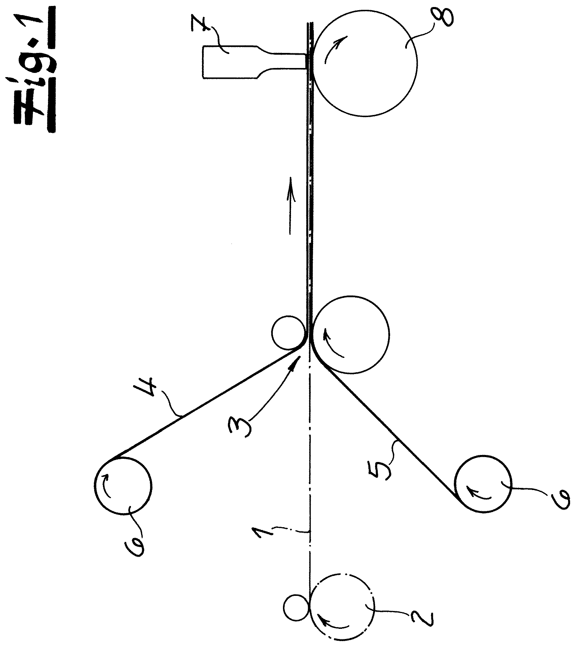

Fig.1 shows in a general manner the production of a stretch-bonded laminate with a stretch-bonding in the machine direction MD. With respect to the specific embodiment the machine direction MD corresponds to the stretch direction of the laminate and the cross direction CD corresponds to the transverse direction of the laminate.

According to fig. 1 an elastic film 1 is provided on a first roll 2. The elastic film 1 runs along the machine direction MD to a nip 3 of a roll arrangement. At the nip 3 the elastic film 1 is combined with a first nonwoven web 4 and a second nonwoven web 5 provided from separate roles 6.

As the circumferential speed of the roll arrangement forming the nip 3 is higher than the circumferential speed of the first roll 2 the elastic film 1 is stretched along the machine direction MD. In difference to the first roll 2 the separate roles 6 providing the first nonwoven web 4 and the second nonwoven web 5 rotate with essentially the same circumferential speed as the roles of the role arrangement forming the nip 3.

The stretching ratio of the elastic film 1 corresponds to the ratio of the circumferential speed of the first roll 2 in comparison to the circumferential speed of the role arrangement at the nip 3.

After the nip 3 and ultrasonic bonding station comprising a sonotrode 7 and an amboss roll 8 are provided. As the amboss roll 8 rotates with nearly the same circumferential speed as the rolls at the nip 3 the elastic film 1 is held in the stretched condition. The stretching rate is typically between 150% and 500%.

The machine direction stretch-bonded laminate can be directly processed or rolled up for subsequent processing.

When the tension is released, the first nonwoven web 4 and the second nonwoven web 5 are gathered due to the elastic relaxation of the elastic film 1.

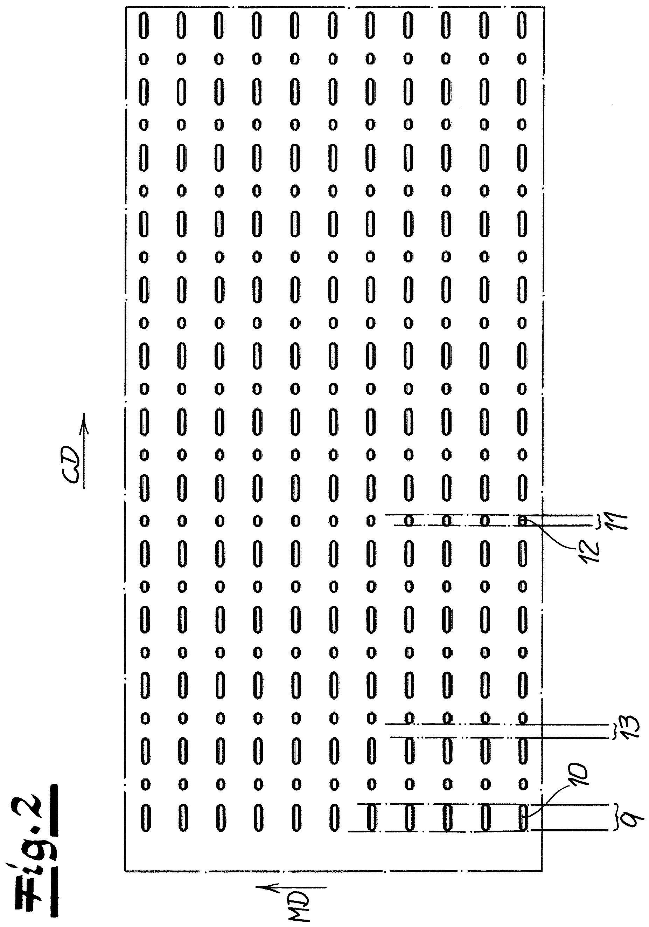

Fig. 2 shows a bonding pattern which is provided by the amboss roll 8.

Resulting from the bonding pattern shown in fig. 2, the stretch-bonded laminate comprises in an alternating sequence along the transverse direction (corresponding to the cross direction CD) first rows 9 of structures 10 elongated along the transverse direction (corresponding to the cross direction). The stretch-bonded laminate further comprises second rows 11 with bonding areas 12.

The first and second rows 9, 11 are separated by lines 13 extending in the stretch direction. At the lines 13 the first nonwoven web 4 and the second nonwoven web 5 lie unconnected on the uninterrupted elastic film 1 after the ultrasonic bonding process.

According to the specific embodiment shown the width of the first rows 9 is about 2 mm, wherein the width of the second rows is about 0.8 mm. With the width of the lines 13 of 0.8 mm the distance between successive first rows (measured edge-to-edge) is about 2.4 mm.

With respect to the shown embodiment the elongated structures 10 and the bonding areas 12 are aligned along columns extending in the transverse direction. The distance between the columns is about 2.5 mm (measured edge-to-edge).

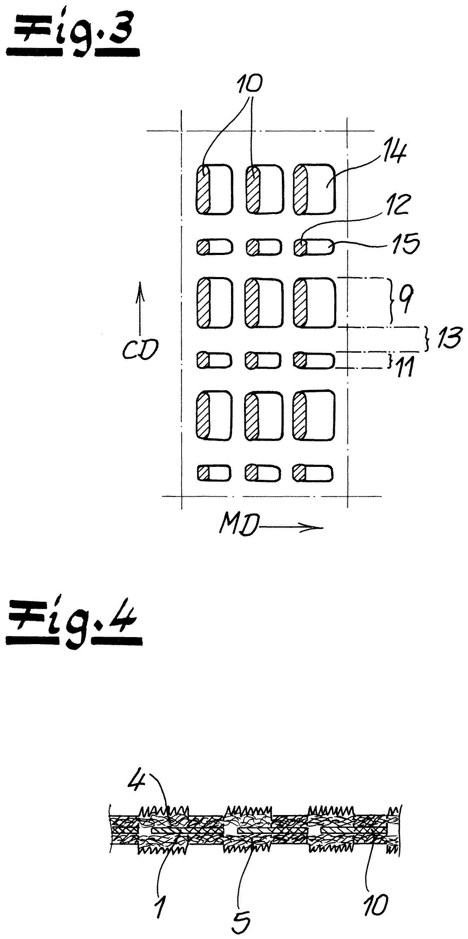

Fig. 3 shows in a simplified manner the structure of the elastic film after the ultrasonic bonding process in the stretched state. At the bonding areas 12 and the elongated structures 10 the first nonwoven web 4 and the second nonwoven web 5 are bonded to the elastic film 1. Due to the elasticity of the stretched elastic film 1 and the melting of the elastic film 1 at the elongated structures 10 the elastic film 1 retracts adjacent to the elongated structure 10 forming a perforation 14. As an alternative, perforations 14 may occur on both sides of each elongated structure 10.

Additional perforations 15 develop adjacent to the bonding areas 12.

Considering fig. 3, it is clear that the elastic properties of the machine direction stretch-bonded laminate are essentially provided by these lines 13 between the first and second rows 9, 11.

Fig. 4 shows a cross-section through the first row 9 of the machine direction stretch-bonded laminate in the relaxed state. Due to the elastic relaxation of the elastic film 1 the first nonwoven web 4 and the second nonwoven web 5 are gathered along the stretching directions so that a clothes-like soft appearance is achieved. In difference to a laminate comprising stretch-bonded elastic strands the bulkiness of the first nonwoven web 4 and the second nonwoven web 5 is limited to an advantageous extent. In addition a good structural stability of the complete stretch-bonded laminate is achieved with respect to the stretch direction and especially the transverse direction.

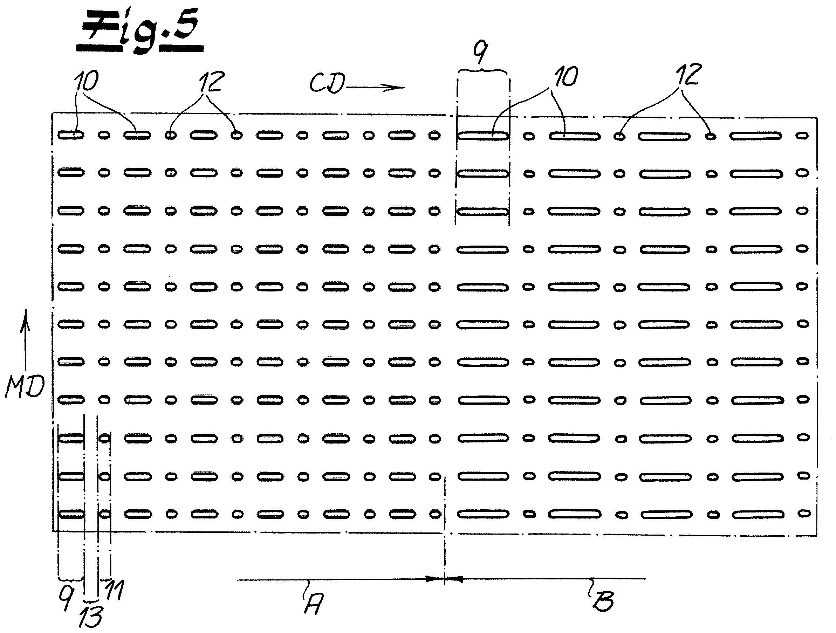

In principle the bonding pattern shown in fig. 2 can be modified along the transverse direction in segments or along the stretch direction in sections. As an example fig. 5 shows a modified bonding pattern with a first segment A extending in the stretch direction with the first rows 9 having a first width and a second segment B extending in the stretch direction with the first rows having a different, larger second width. Only by changing the width of the first rows 9 the stretching properties can be varied easily. At the second segment B the elastic forces are lower than at the first segment A as the larger width of the elongated structures 10 in the second segment B lead to an enhanced weakening of the elastic film 1.

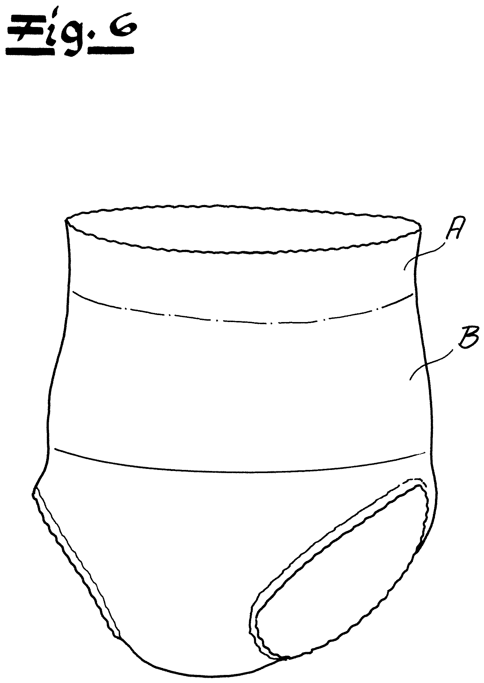

Fig. 6 shows a training pant with the machine direction stretch-bonded laminate extending around the hip region. To provide a different tension at the upper end of the training pant and below different segments A, B as shown in fig. 5 can be provided.

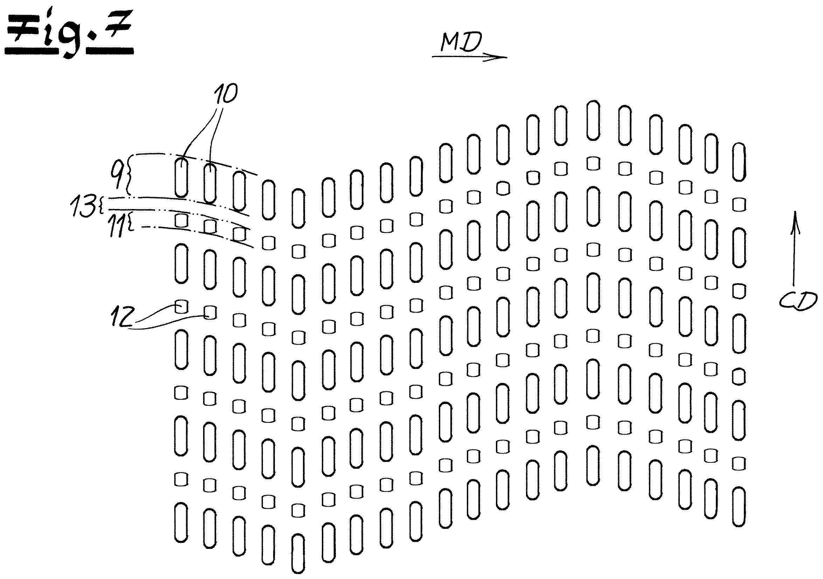

Fig. 7 shows as an alternative a bonding pattern with the first rows 9, the second rows 11 and the lines 13 extending wavelike in the stretch direction. This alternative embodiment is essentially provided for esthetic reasons without changing the basic features of the present invention.

## Claims

1. Stretch-bonded laminate with an elastic film (1) and at least one first nonwoven web (4) comprising a stretch direction along which the first nonwoven web (4) is gathered due to the elastic relaxation of the elastic film (1) and a transverse direction, further comprising in an alternating sequence along the transverse direction elongated structures (10) forming first rows (9) and and bonding areas (12) forming second rows (11) extending in the stretch direction,
wherein said elongated structures (10) are elongated along an angle of less than 5° to the transverse direction with the elongated structures (10) being attributed to perforations (14) of the elastic film (1),
wherein the elongated structures (10) and the bonding areas (12) are together aligned along columns extending in the transverse direction wherein the first and second rows (9, 11) are separated by lines (13) extending in the stretch direction with the first nonwoven web (4) lying unconnected on the uninterrupted elastic film (1),
wherein the width of the first rows (9) is more than 1.5 of the width of the second rows (11) measured in the transverse direction,
wherein the distance between successive first rows (9) is between 1 mm and 5 mm.

2. Stretch-bonded laminate according to claim 1, wherein the first rows (9), the second rows (11) and the lines (13) extend straight in the stretch direction.

3. Stretch-bonded laminate according to claim 1, wherein the first rows (9), the second rows (11) and the lines (13) extend wavelike in the stretch direction with a maximum deviation angle below 45°.

4. Stretch-bonded laminate according to claim 1, wherein the elongated structures (10) are embodied as bond lines with an adjoining perforation (14) on at least one side.

5. Stretch-bonded laminate according to claim 1, wherein the elastic film (1) is provided between the first nonwoven web (4) and a second nonwoven web (5).

6. Stretch-bonded laminate according to claim 1, wherein in at a predetermined threshold stretching rate the first nonwoven web (4) lies flat on the elastic film (1) without deformation of its initial structure.

7. Stretch-bonded laminate according to claim 6, wherein the predetermined threshold stretching rate corresponds to a stretching rate of 100% to 500%.

8. Stretch-bonded laminate according to claim 6 or 7, wherein with respect to the predetermined threshold stretching rate the area ratio of the elongated structures (10) is between 12% and 4%.

9. Stretch-bonded laminate according to claim 1, wherein the width of the lines (13) is between 0.4 mm and 1.6 mm.

10. Stretch-bonded laminate according to claim 1, wherein the width of the first rows (9) is between 1 mm und 5 mm.

11. Stretch-bonded laminate according to claim 1, wherein the width of the second rows (11) is between 0.4 mm and 1.6 mm.

12. Stretch-bonded laminate according to claim 1, wherein additional perforations (15) are provided adjoining to the bonding areas.

13. Stretch-bonded laminate according to claim 1, wherein in a first segment (A) of the laminate extending in the stretch direction the first rows (9) have a first width and in a second segment (B) of the laminate extending in the stretch direction the first rows (9) have a different second width.

14. Stretch-bonded laminate according to claim 1, comprising along the stretch direction first sections with the first rows (9), the second rows (11) and the lines (13) in between, wherein at the second sections the elongated structures (10) and/or the bonding areas (12) are omitted and/or provided in a different pattern.

15. Method to produce a machine direction stretch-bonded laminate according to one of the claims 1 to 14, wherein
a. a continuous web of an elastic film (1) is transported and stretched along a machine direction (MD) with a stretching ratio between 100% and 500%,
b. at least a first nonwoven web (4) is transported along the machine direction (MD),
c. the elastic film (19 and the first nonwoven web (4) are laminated by ultrasonic bonding in a bonding pattern with the elastic film (1) held in the stretched state,
d. the bonding pattern comprises in an alternating sequence perpendicular to the machine direction (MD) along a cross direction (CD) first rows (9) and second rows (11) extending in the machine direction (MD),
e. said first rows (9) comprise structures (10) elongated along an angle of less than 5° to the cross direction (CD),
f. due to the stretching of the elastic film (1) and the ultrasonic bonding at least one perforation (14) of the elastic film (1) forms adjacent to each elongated structure (10),
g. said second rows comprise (11) bonding areas (12),
h. the first and second rows (9, 11) are separated by lines (13) extending in the machine direction (MD) with the first nonwoven web (4) lying unconnected on the uninterrupted elastic film (1),
i. the width of the first rows (9) is more than 1.5 of the width of the second rows (11) measured in the cross direction (CD) and
j. the distance between successive first rows (9) is between 1 mm and 5 mm.

16. Absorbent article including a chassis comprising a topsheet, a backsheet, and an absorbent core disposed between the topsheet and the backsheet, and further including a stretch-bonded laminate according to one of the claims 1 to 14 as elastic belt fixed to the chassis.

17. Absorbent article according to claim 16, wherein the elastic belt is fixed essentially over a full overlapping surface of the belt and backsheet.

18. Absorbent article according to claim 16, wherein the elastic belt is fixed over a portion of less than 80% of overlapping areas of belt and backsheet.

## Patentansprüche

1. Streckgebundenes Laminat mit einer elastischen Folie (1) und mindestens einem ersten Vliesstoffgewebe (4), das eine Streckrichtung, entlang derer das erste Vliesstoffgewebe (4) wegen der elastischen Relaxation der elastischen Folie (1) gerafft ist, und eine Querrichtung aufweist, das weiterhin in einer abwechselnden Reihenfolge entlang der Querrichtung längliche Strukturen (10), die erste Reihen (9) bilden, und Verbindungsbereiche (12) aufweist, die sich in der Streckrichtung erstreckende zweite Reihen (11) bilden,
wobei die länglichen Strukturen (10) sich längs entlang einem Winkel von kleiner als 5° zur Querrichtung erstrecken, wobei die länglichen Strukturen (10) Perforationen (14) der elastischen Folie (1) zugeordnet sind,
wobei die länglichen Strukturen (10) und die Verbindungsbereiche (12) zusammen entlang sich in der Querrichtung ersteckenden Säulen ausgerichtet sind,
wobei die ersten und zweiten Reihen (9, 11) durch Zeilen (13) getrennt sind, die sich in der Streckrichtung erstrecken, wobei das erste Vliesstoffgewebe (4) ungebunden auf der ununterbrochenen elastischen Folie (1) aufliegt,
wobei die Breite der ersten Reihen (9) größer als 1,5-mal der Breite der zweiten Reihen (11) ist, in der Querrichtung gemessen,
wobei der Abstand zwischen aufeinander folgenden ersten Reihen (9) zwischen 1 mm und 5 mm beträgt.

2. Streckgebundenes Laminat nach Anspruch 1, wobei die ersten Reihen (9), die zweiten Reihen (11) und die Zeilen (13) sich in der Streckrichtung gerade erstrecken.

3. Streckgebundenes Laminat nach Anspruch 1, wobei die ersten Reihen (9), die zweiten Reihen (11) und die Zeilen (13) sich in der Streckrichtung wellenartig mit einem maximalen Abweichungswinkel kleiner als 45° erstrecken.

4. Streckgebundenes Laminat nach Anspruch 1, wobei die länglichen Strukturen (10) als Verbindungslinien mit einer benachbarten Perforation (14) auf mindestens einer Seite ausgeführt sind.

5. Streckgebundenes Laminat nach Anspruch 1, wobei die elastische Folie (1) zwischen dem ersten Vliesstoffgewebe (4) und einem zweiten Vliesstoffgewebe (5) vorgesehen ist.

6. Streckgebundenes Laminat nach Anspruch 1, wobei bei einer vorab bestimmten Schwelle für die Streckrate das erste Vliesstoffgewebe (4) eben auf der elastischen Folie (1) ohne Verformung aus seiner ursprünglichen Struktur aufliegt.

7. Streckgebundenes Laminat nach Anspruch 6, wobei die vorab bestimmte Schwelle für die Streckrate einer Streckrate von 100 % bis 500 % entspricht.

8. Streckgebundenes Laminat nach Anspruch 6 oder 7, wobei mit Bezug auf die vorab bestimmte Schwelle für die Streckrate das Flächenanteil der länglichen Strukturen (10) zwischen 12 % und 4 % liegt.

9. Streckgebundenes Laminat nach Anspruch 1, wobei die Breite der Zeilen (13) zwischen 0,4 mm und 1,6 mm ist.

10. Streckgebundenes Laminat nach Anspruch 1, wobei die Breite der ersten Reihen (9) zwischen 1 mm und 5 mm ist.

11. Streckgebundenes Laminat nach Anspruch 1, wobei die Breite der zweiten Reihen (11) zwischen 0,4 mm und 1,6 mm ist.

12. Streckgebundenes Laminat nach Anspruch 1, wobei zusätzliche Perforationen (15) benachbart zu den Verbindungsbereichen vorgesehen sind.

13. Streckgebundenes Laminat nach Anspruch 1, wobei in einem ersten Segment (A) des Laminats, das sich in der Streckrichtung erstreckt, die ersten Reihen (9) eine erste Breite aufweisen, und in einem zweiten Segment (B) des Laminats, das sich in der Streckrichtung erstreckt, die ersten Reihen (9) eine unterschiedliche zweite Breite aufweisen.

14. Streckgebundenes Laminat nach Anspruch 1, das entlang der Streckrichtung erste Abschnitte mit den ersten Reihen (9), den zweiten Reihen (11) und den Zeilen (13) dazwischen aufweist, wobei an den zweiten Abschnitten die länglichen Strukturen (10) und/oder die Verbindungsbereiche (12) weggelassen sind und/oder in einem unterschiedlichen Muster vorgesehen sind.

15. Verfahren zum Erzeugen eines in Maschinenrichtung streckgebundenen Laminats nach einem der Ansprüche 1 bis 14, wobei
a. ein durchgehendes Gewebe einer elastischen Folie (1) entlang einer Maschinenrichtung (MD) transportiert und mit einem Streckverhältnis zwischen 100 % und 500 % gestreckt wird,
b. mindestens ein erstes Vliesstoffgewebe (4) entlang der Maschinenrichtung (MD) transportiert wird,
c. die elastische Folie (19) und das erste Vliesstoffgewebe (4) durch Ultraschallschweißen in einem Verbundmuster mit der im gestreckten Zustand gehaltenen elastischen Folie (1) laminiert werden,
d. das Verbundmuster in einer abwechselnden Reihenfolge senkrecht zur Maschinenrichtung (MD) entlang einer Querrichtung (CD) erste Reihen (9) und zweite Reihen (11) aufweist, die sich in der Maschinenrichtung (MD) erstrecken,
e. die ersten Reihen (9) Strukturen (10) aufweist, die in einem Winkel kleiner als 5° zur Querrichtung (CD) längserstreckt sind,
f. wegen des Streckens der elastischen Folie (1) und des Ultraschallschweißens sich mindestens eine Perforation (14) der elastischen Folie (1) benachbart zu jeder länglichen Struktur (10) bildet,
g. die zweiten Reihen (11) Verbindungsbereiche (12) aufweisen,
h. die ersten und zweiten Reihen (9, 11) durch Zeilen (13) getrennt sind, die sich in der Maschinenrichtung (MD) erstrecken, wobei das erste Vliesstoffgewebe (4) ungebunden auf der ununterbrochenen elastischen Folie (1) aufliegt,
i. die Breite der ersten Reihen (9) größer als 1,5-mal der Breite der zweiten Reihen (11) ist, in der Querrichtung gemessen,
j. der Abstand zwischen aufeinander folgenden ersten Reihen (9) zwischen 1 mm und 5 mm beträgt.

16. Absorptionsfähiger Artikel umfassend ein Chassis, welches eine obere Lage, eine hintere Lage und einen absorptionsfähigen Kern zwischen der oberen Lage und der hinteren Lage aufweist und weiterhin ein streckgebundenes Laminat nach einem der Ansprüche 1 bis 14 als an dem Chassis befestigtes elastisches Band aufweist.

17. Absorptionsfähiger Artikel nach Anspruch 16, wobei das elastische Band im Wesentlichen über einer vollständigen überlappenden Fläche des Bands und der hinteren Lage befestigt ist.

18. Absorptionsfähiger Artikel nach Anspruch 16, wobei das elastische Band über einem Abschnitt von weniger als 80 % der überlappenden Flächen des Bands und der hinteren Lage befestigt ist.

## Revendications

1. Stratifié lié par étirement avec un film élastique (1) et au moins un premier voile non tissé (4) comprenant une direction d'étirement le long de laquelle le premier voile non tissé (4) est rassemblé en raison de la relaxation élastique du film élastique (1) et une direction transversale, comprenant en outre dans une séquence alternée le long de la direction transversale des structures allongées (10) formant des premières rangées (9) et des zones de liaison (12) formant des deuxièmes rangées (11) s'étendant dans la direction d'étirement,
dans lequel lesdites structures allongées (10) sont allongées selon un angle inférieur à 5° par rapport à la direction transversale, les structures allongées (10) étant attribuées aux perforations (14) du film élastique (1),
dans lequel les structures allongées (10) et les zones de collage (12) sont alignées ensemble le long de colonnes s'étendant dans la direction transversale,
dans lequel les premières et deuxièmes rangées (9, 11) sont séparées par des lignes (13) s'étendant dans le sens de l'étirement avec le premier voile non tissé (4) reposant sans connexion sur le film élastique ininterrompu (1),
dans lequel la largeur des premières rangées (9) est supérieure à 1,5 fois la largeur des deuxièmes rangées (11) mesurée dans la direction transversale,
dans lequel la distance entre les premières rangées successives (9) est comprise entre 1 mm et 5 mm.

2. Stratifié lié par étirement selon la revendication 1, dans lequel les premières rangées (9), les deuxièmes rangées (11) et les lignes (13) s'étendent de façon rectiligne dans la direction de l'étirement.

3. Stratifié lié par étirement selon la revendication 1, dans lequel les premières rangées (9), les deuxièmes rangées (11) et les lignes (13) s'étendent en forme de vague dans la direction d'étirement avec un angle de déviation maximum inférieur à 45°.

4. Stratifié lié par étirement selon la revendication 1, dans lequel les structures allongées (10) sont représentées par des lignes de liaison avec une perforation adjacente (14) sur au moins un côté.

5. Stratifié lié par étirement selon la revendication 1, dans lequel le film élastique (1) est placé entre le premier voile non tissé (4) et un deuxième voile non tissé (5).

6. Stratifié lié par étirement selon la revendication 1, dans lequel, à un taux d'étirement de seuil prédéterminé, le premier voile non tissé (4) s'étend à plat sur le film élastique (1) sans déformation de sa structure initiale.

7. Stratifié lié par étirement selon la revendication 6, dans lequel le taux d'étirement de seuil prédéterminé correspond à un taux d'étirement compris entre 100 % et 500 %.

8. Stratifié lié par étirement selon la revendication 6 ou la revendication 7, dans lequel, par rapport au taux d'étirement de seuil prédéterminé, le rapport de surface des structures allongées (10) est compris entre 12 % et 4 %.

9. Stratifié lié par étirement selon la revendication 1, dans lequel la largeur des lignes (13) est comprise entre 0,4 mm et 1,6 mm.

10. Stratifié lié par étirement selon la revendication 1, dans lequel la largeur des premières rangées (9) est comprise entre 1 mm et 5 mm.

11. Stratifié lié par étirement selon la revendication 1, dans lequel la largeur des deuxièmes rangées (11) est comprise entre 0,4 mm et 1,6 mm.

12. Stratifié lié par étirement selon la revendication 1, dans lequel des perforations supplémentaires (15) sont prévues à côté des zones de collage.

13. Stratifié lié par étirement selon la revendication 1, dans lequel dans un premier segment (A) du stratifié s'étendant dans le sens de l'étirement, les premières rangées (9) ont une première largeur et dans un deuxième segment (B) du stratifié s'étendant dans le sens de l'étirement, les premières rangées (9) ont une deuxième largeur différente.

14. Stratifié lié par étirement selon la revendication 1, comprenant le long de la direction d'étirement des premières sections avec les premières rangées (9), les deuxièmes rangées (11) et les lignes (13) entre elles, dans lequel dans les deuxièmes sections les structures allongées (10) et/ou les zones de liaison (12) sont omises et/ou fournies selon un motif différent.

15. Procédé de fabrication d'un stratifié lié par étirement dans le sens machine selon l'une quelconque des revendications 1 à 14, dans lequel
a. un voile continu d'un film élastique (1) est transporté et étiré le long de la direction machine (MD) avec un taux d'étirement compris entre 100 % et 500 %,
b. au moins un premier voile non tissé (4) est transporté le long de la direction machine (MD),
c. le film élastique (19) et le premier voile non tissé (4) sont laminés par collage ultrasonique selon un schéma de collage, le film élastique (1) étant maintenu à l'état étiré,
d. le motif de collage comprend, dans une séquence alternée perpendiculaire à la direction machine (MD) le long d'une direction transversale (CD), des premières rangées (9) et des deuxièmes rangées (11) s'étendant dans la direction machine (MD),
e. lesdites premières rangées (9) comprennent des structures (10) allongées selon un angle inférieur à 5° par rapport à la direction transversale (CD),
f. en raison de l'étirement du film élastique (1) et du collage par ultrasons, au moins une perforation (14) du film élastique (1) se forme à côté de chaque structure allongée (10),
g. lesdites deuxièmes rangées comprennent (11) des zones de collage (12),
h. les premières et les deuxièmes rangées (9, 11) sont séparées par des lignes (13) s'étendant dans la direction machine (MD) avec le premier voile non tissé (4) reposant non connecté sur le film élastique ininterrompu (1),
i. la largeur des premières rangées (9) est supérieure à 1,5 fois la largeur des deuxièmes rangées (11) mesurée dans le sens transversal (CD) et
j. la distance entre les premières rangées successives (9) est comprise entre 1 mm et 5 mm.

16. Article absorbant comprenant un châssis composé d'un feuillet supérieur, d'un feuillet postérieur et d'un noyau absorbant disposé entre le feuillet supérieur et le feuillet postérieur, et comprenant en outre un stratifié lié par étirement selon l'une quelconque des revendications 1 à 14 en tant que courroie élastique fixée au châssis.

17. Article absorbant selon la revendication 16, dans lequel la ceinture élastique est attachée essentiellement sur une surface de chevauchement complète de la ceinture et du feuillet postérieur.

18. Article absorbant selon la revendication 16, dans lequel la courroie élastique est attachée sur une portion représentant moins de 80 % des zones de chevauchement de la courroie et du feuillet postérieur.
